# EUROPEAN PATENT APPLICATION

(11) **EP 4 506 092 A1**
(43) Date of publication of application: **12.02.2025**
(21) Application number: 24761483.7
(22) Date of filing: 09.01.2024
(51) Int. Cl.: B23B 51/00

(54) **DRILL**

(30) Priority: 23.06.2023 JP 2023103018
(71) Applicant: Medmetalex Co., Ltd, Nishiyodogawa-ku Osaka-shi, Osaka 555-0012 (JP)
(72) Inventor: YAMAUCHI Yuji, Osaka-shi, Osaka 555-0012 (JP)
(74) Representative: VKK Patentanwälte PartG mbB
(86) International application number: PCT/JP2024/000137
(87) International publication number: WO 2024/262063

(57) **Abstract**

Problem: To provide a drill with good cutting quality and excellent durability.

Means for Solution: A drill comprises a chip discharging groove 6 formed on a periphery of a drill body 2 rotating around an axis, a first thinning face 10 formed from the outer circumference of the drill body across the chip discharging groove and oblique up on the tip portion, a first relief surface 11 that forms a first cutting edge 14 that is the main cutting edge on the boundary ridge line with the first thinning face and is formed on the opposite side of the drill rotation direction, in contact with the first thinning face, a second thinning face 12 formed on the tip portion of the first thinning face, in contact with the first thinning face and opposite the direction of drill rotation, and second cutting edge 15 formed on the boundary ridge line between the second thinning face and the first relief surface. The first cutting edge on the outer circumference side reaches the outer circumferential surface of the drill or the vicinity of the outer circumferential surface of the drill.

## Description

This invention relates to drills in general and to medical drills in particular.

Drills used for drilling are used in numerous industries. Drills used in surgical operations are often used for drilling bone. Bone is covered with hard cortical bone on the surface and soft trabecular bone inside. Cortical bone is very hard, with a Shore hardness of over 90, which corresponds to a Vickers hardness of 820 Hv and a Rockwell hardness of 64.7 HRC.

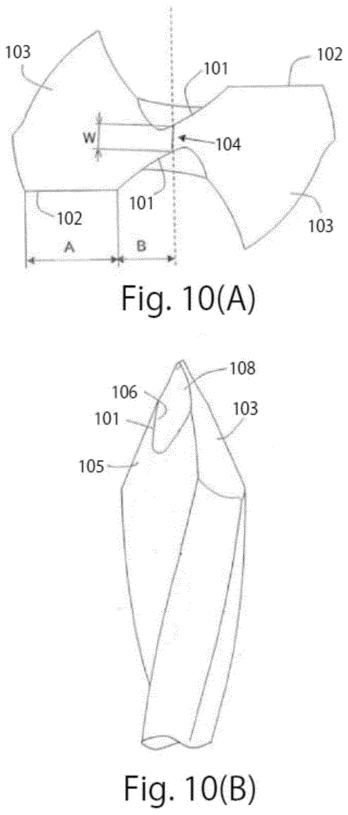

The drill shown in Fig. 10 is a surgical operation drill, the purpose of which is to provide a medical drill that is resistant to wear and has excellent cutting ability by improving the cutting force and the cutting ability of the cutting edge created from the inner end of the tip portion to the outer circumference.

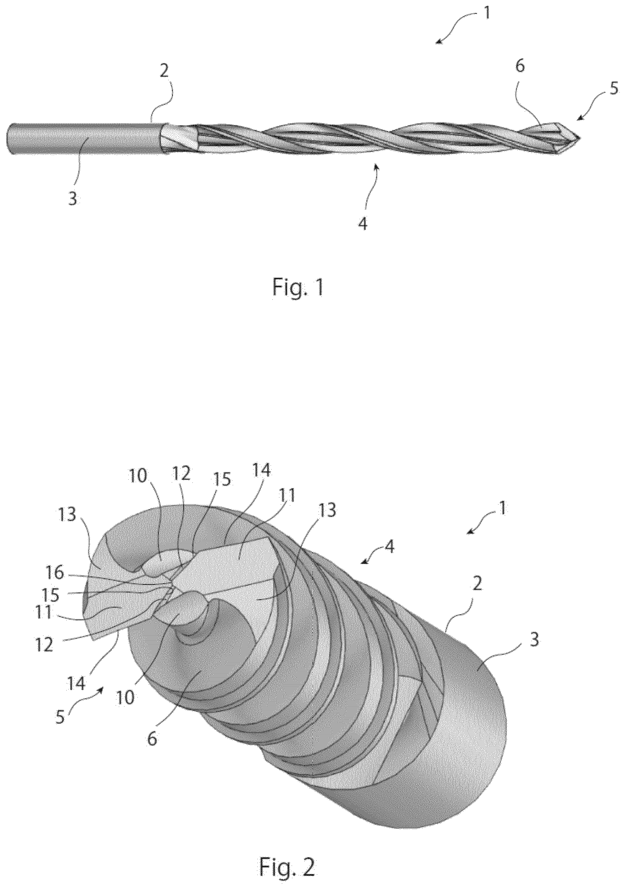

Figure 10 is the equivalent of Figure 2 of Patent Documents 1. Figure 10(a) is a plan view from the tip side, and Figure 10(b) is a side view of the tip portion. A thinning cutting edge 101 is formed on the tip portion. The thinning cutting edge 101 is formed at the boundary between the rake face 106 and the relief surface 103 formed by thinning. The main cutting edge 102 is continuous with the thinning cutting edge 101 and is formed on the periphery of the thinning cutting edge 101. A thinning face 108 is formed from the rake face 105 formed by the main cutting edge and the thinning cutting edge 101. A chisel 104 is formed between the two thinning cutting edges 101.

### [Prior Art Documents] Patent Publication JP2018-108223

In the medical drill described in Patent Documents 1, there are only two thinning faces 108 on the shaft object that form the 101 thinning cutting edge, and when the thinning recess is enlarged to improve cutting quality, the center thickness becomes smaller, reducing the rigidity of the drill, and damage to the drill cannot be avoided. Generally, drills are used repeatedly and therefore need to be durable, but the drill described in the cited reference 1 has durability problems.

The present invention was made in view of these points, and its purpose is to provide a drill with good cutting quality and excellent durability.

The invention (1) comprises a chip discharging groove formed on a periphery of a drill body rotating around an axis;
a first thinning face formed from the outer circumference of the drill body across the chip discharging groove and oblique up on the tip portion;
a first relief surface that forms a first cutting edge that is the main cutting edge on the boundary ridge line with the first thinning face and is formed on the opposite side of the drill rotation direction, in contact with the first thinning face;
a second thinning face formed on the tip portion of the first thinning face, in contact with the first thinning face and opposite the direction of drill rotation;
And a second cutting edge formed on the boundary ridge line between the second thinning face and the first relief surface; and, wherein the first cutting edge on the outer circumference side reaches the outer circumferential surface of the drill or the vicinity of the outer circumferential surface of the drill.

In the invention (1), the first and second thinning faces are provided in the axial direction, which allows for a larger center thickness. As a result, the cutting quality and durability of the drill can be increased. In addition, since the outer circumferential edge of the first cutting edge reaches the outer circumferential surface of the drill or the vicinity of the outer circumferential surface of the drill, a constant cutting quality can be maintained at any position of the cutting edge.

The invention (2) is characterized in that a second rake angle of the second cutting edge by the second thinning face is a positive angle.

In the invention (2), the second rake angle of the second cutting edge by the second thinning face is a positive angle, which reduces drill misalignment when drilling at an angle to the perforated body. This is especially desirable when drilling hard objects such as cortical bone.

The invention (3) is the drill of the invention (2), wherein the second rake angle is 20° to 60°. If the rake angle is too small, the cutting edge strength becomes small, and if the rake angle is too large, the drill tip portion becomes slippery. After examining various angles, 20° is preferable for the lower limit, 30° is even more preferable, and 35° is even more preferable. For the upper limit, 60° is preferred, 50° is more preferred, and 45° is even more preferred.

The invention (4) is the drill of any of the inventions (1) through (3), wherein the length of the chisel edge formed on the tip portion is zero. The tip is sharp because there is virtually no chisel edge, and when the drill tip is placed against cortical bone and rotated, the initial bite is even better and more resistant to displacement.

According to this invention, a drill with good cutting quality and excellent durability can be provided.

### Brief description of the drawings

Figure 1: Side view of the entire drill of Embodiment 1 of this invention.
Figure 2: Oblique drawing of the entire drill of Embodiment 1 of this invention.
Figure 3: Oblique drawing showing the drill tip portion of Embodiment 1 of this invention.
Figure 4: This schematic illustrates the positive and negative rake angles of a drill.
Figure 5: Side view of the entire drill of Embodiment 2 of this invention.
Figure 6: Oblique drawing showing the drill tip portion of Embodiment 2 of this invention.
Figure 7: This photograph shows the condition of the tip portion after a drilling test using the drill of Embodiment1 of this invention.
[Figure 8] This is a photograph showing the condition of the tip portion after a drilling test using the drill described in Patent Document 1.
[Figure 9]: This figure shows the drilling test results of the drill of Embodiment 1 and the drill described in Patent Document 1.
[Figure 10] This figure shows the structure of the drill described in Patent Document 1.

The following description of Embodiments of the invention will be described in detail on the basis of the Drawings. The following Description of Embodiments is illustrative in nature and is not intended to limit the scope of the invention.

Figure 1 shows a side view of the entire drill of Embodiment 1. Drill 1 has a shank portion 3 formed at one end of a drill body 2 and a cutting portion 4. The cutting portion 4 has a chip discharging groove 6 formed from the tip portion to the shank portion 3 and connected to the cutting edge portion 5.

Figure 2 is an oblique drawing of the entire drill of Embodiment 1. Drill 1 has a shank portion 3 formed at one end of drill body 2 and a cutting portion 4, but the invention is characterized by the shape of the cutting edge portion 5. A first cutting edge 14 is formed at the boundary between the first thinning face 10 and the first relief surface 11. A second relief surface 13 is formed on the opposite side of the first relief surface 11 in the direction of rotation.

A second thinning face 12 is formed on the tip portion of the first thinning face 10, in contact with the first thinning face 10 and opposite to the direction of drill rotation. A second cutting edge 15 is formed on the boundary ridge line between the second thinning face 12 and the first relief surface 11.

A chisel edge 16 is formed between two opposing first relief surfaces 11. The second relief surface 13 is connected to the chip discharging groove 6.

The first cutting edge 14 is connected to the outer periphery of the drill, and the cutting edges are the first cutting edge 14 and the second cutting edge 15. However, it is not necessary to be limited to this form, as in the drill of Patent Document 1, the chip discharging groove 6 can be a rake face and a relief surface can be formed on the outer periphery of the drill to provide the main cutting edge on the periphery of the first cutting edge 14. The main cutting edge may be formed on the outer periphery of the first cutting edge 14.

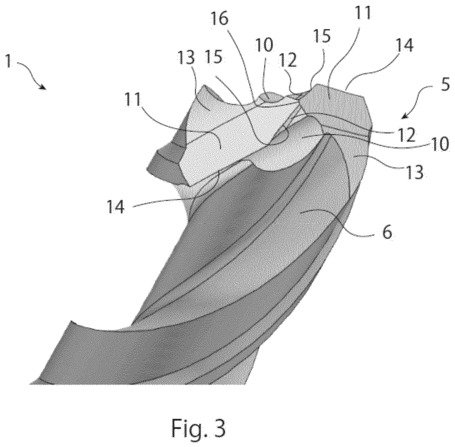

Figure 3 is an enlarged oblique drawing of the cutting edge portion 5 of the tip portion of the drill 1 of Embodiment 1 shown in Figure 2. The chip discharging groove 6, first thinning face 10, first relief surface 11, second thinning face 12, second relief surface 13, first cutting edge 14, second cutting edge 15, and chisel edge 16 are as shown in the figure and are not explained.

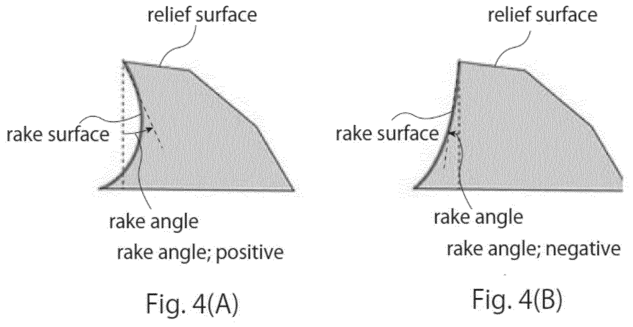

Figure 4 is a schematic illustration of the positive and negative rake angles of a drill. Figure 4(A) shows the case where the rake angle is positive angle, and Figure 4(B) shows the case where the rake angle is negative angle. When the relationship between the rake face and the relief surface is Figure 4(A), the rake angle = positive angle, and when Figure 4(B), the rake angle = negative angle. When the rake angle is positive angle, the cutting edge is sharp and the cutting edge bites sharply into the perforated body, making it more resistant for the drill edge to slip.

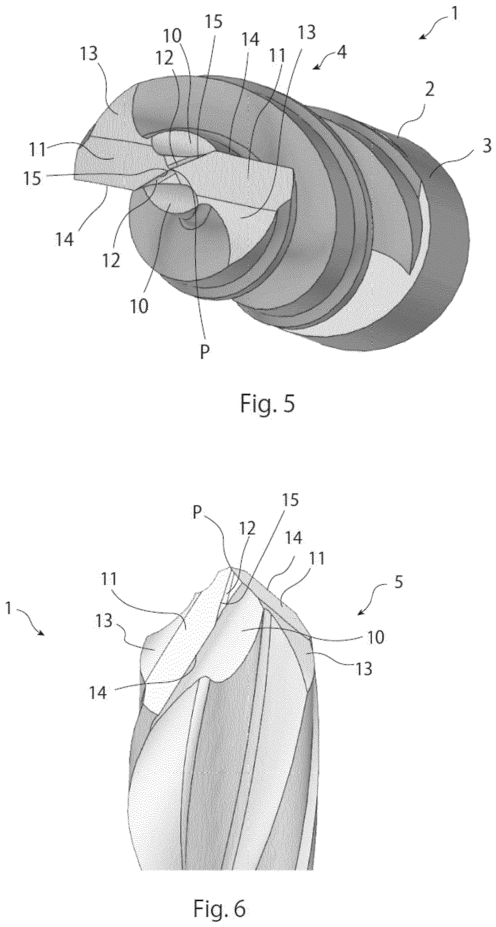

Figure 5 is an oblique drawing of the entire drill of Embodiment 2 of this invention. The drill has almost the same structure as the drill of Embodiment 1 shown in Figure 2, but the drill of Embodiment 1 had a chisel edge 16 at the tip portion of the drill, while the drill of Embodiment 2 shown in Figure 5 has no chisel edge and the tip is point P.

The chisel edge increases the wall thickness formed by the second thinning face 12, but when the chisel edge is removed, instead of a smaller wall thickness at the tip portion, the tip sharply penetrates the perforated body for better cutting quality. Other parts are the same as in Figure 2, so the explanation is omitted.

Figure 6 is an oblique drawing showing the drill tip of Embodiment 2. The description of Embodiment 2 is omitted because it is the same as the drill of Embodiment 1 shown in Figure 3 except that no chisel edge is formed on the tip portion P.

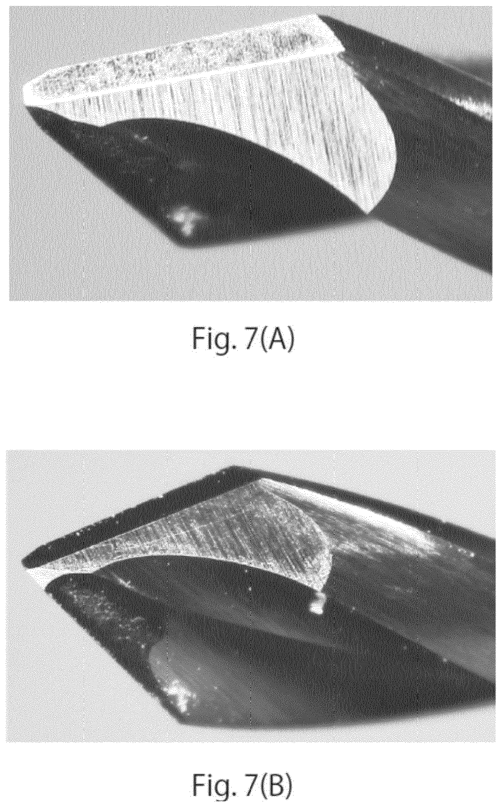

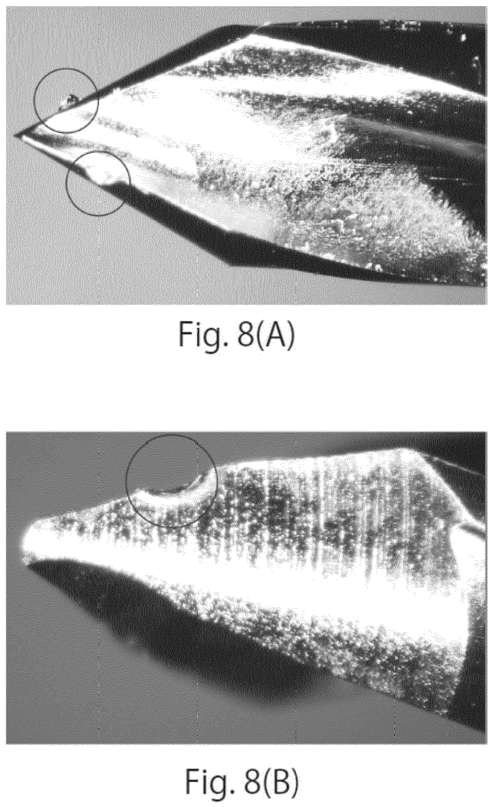

Figures 7(A) and 7(B) show the state of the tip portion after a drilling test using the drill of Embodiment 1 (Example), and Figures 8(A) and 8(B) show the state of the tip portion after a drilling test using the drill described in Patent Documents 1 (Comparative Example). The drilling test conditions are shown below.

### Drilling test conditions

(1) Drill geometry; Both the example and the comparison example have a diameter of 3.2 mm. The center thickness of the example was 1.2 mm, and that of the comparison example was 0.8 mm.
(2) Perforated body; cortical bone sheet (SAW3401-06) 10 mm thick.
(3) Rotation speed; 760RPM
(4) Drill pressing force; 30 N

Figure 7(A) is a photograph of the cutting edge after the drilling test of Example 1, and no damage was observed. Figure 7(B) is a photograph of the cutting edge on the opposite side of Figure 7(A), and this photograph also showed no damage.

Figure 8(A) is a photograph of the cutting edge after the drilling test of the comparative example, and the circled area shows damage from blade flipping. Figure 8(B) is a photograph of the cutting edge on the opposite side of Figure 8(A), showing large depression damage to the cutting edge. This damage was located 0.84 mm from the tip portion and was 0.5 mm in length. Based on these observations, the drill in the example of this invention seems to be very durable compared to the comparative example.

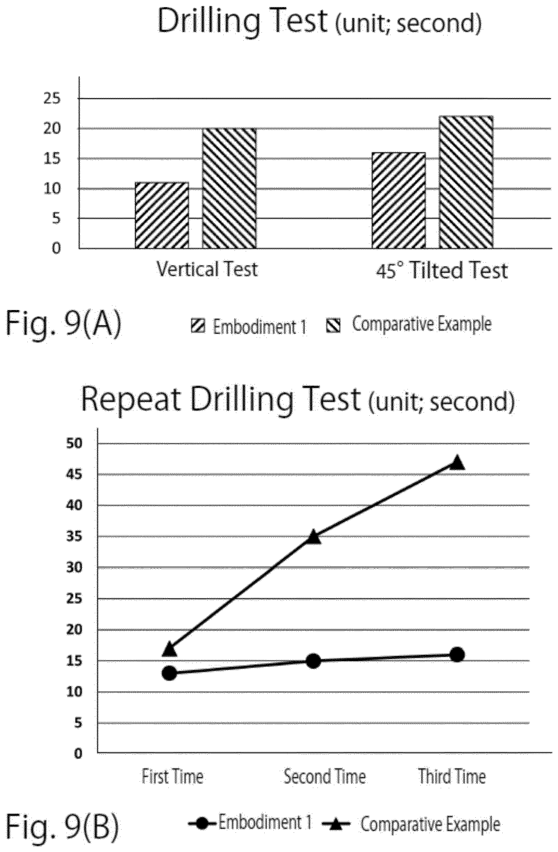

Figure 9(A) shows the results of a drilling test conducted under the above conditions. The drilling work time of the drill of Embodiment 1 of the invention was 11 seconds, while the comparative example described in Patent Documents took 20 seconds, a large difference.

The test results were obtained from a drilling test when the drill was tilted at a 45° angle. The drilling work time of the drill of Embodiment 1 of the invention was 16 seconds, while the comparative example described in Patent Document took 22 seconds, a large difference. In addition, the slip distance of the drill when tilted 45° was 4 mm for the drill of Embodiment 1 of the invention, and 8 mm for the comparative example described in Patent Documents, showing a clear difference.

Figure 9(B) shows the test results when the drilling test was repeated (three times) under the above conditions. The drilling work time of the drill of Embodiment 1 did not change much between 13 and 16 seconds, while that of the comparative example described in Patent Document 1 changed significantly from 17 to 47 seconds, indicating that the durability of the drill of Embodiment 1 is much better than that of the comparative example.

The reason why the durability of the drill of Embodiment 1 of the invention is better than that of the comparative example can be attributed to the fact that damage to the cutting edge portions was hardly observed in the drill of Embodiment 1 of the invention shown in Figure 7, whereas in the comparative example damage occurred after one drilling test as shown in Figure 8.

As explained above, the drill of the invention can be used suitably as a drill with good cutting quality and excellent durability, for example, in surgical operations for drilling bones.

### Reference Signs List

1; drill
2; drill body
3; shank portion
4; cutting edge portion
5; cutting edge portion
6; chip discharging groove
10; first thinning face
11; first relief surface
12; second thinning face
13; second relief surface
14; first cutting edge
15; second cutting edge
16; chisel edge
P; tip portion

## Claims

1.A drill comprising:
a chip discharging groove formed on a periphery of a drill body rotating around an axis;
a first thinning face formed from the outer circumference of the drill body across the chip discharging groove and oblique up on the tip portion;
a first relief surface that forms a first cutting edge that is the main cutting edge on the boundary ridge line with the first thinning face and is formed on the opposite side of the drill rotation direction, in contact with the first thinning face;
a second thinning face formed on the tip portion of the first thinning face, in contact with the first thinning face and opposite the direction of drill rotation;
a second cutting edge formed on the boundary ridge line between the second thinning face and the first relief face; and, wherein the first cutting edge on the outer circumference side reaches the outer circumferential surface of the drill or the vicinity of the outer circumferential surface of the drill.

2. The drill according to claim 1, wherein a second rake angle formed by the second thinning face of the second cutting edge is a positive angle.

3. The drill according to claim 2, wherein the second rake angle is between 20° and 60°.

4. The drill according to any one of claims 1 to 3, wherein a chisel edge formed on the tip portion has a length of zero.
